# EUROPEAN PATENT APPLICATION

(11) **EP 2 422 691 A1**
(43) Date of publication of application: **29.02.2012**
(21) Application number: 10767030.9
(22) Date of filing: 19.04.2010
(51) Int. Cl.: A61B 3/11, A61B 3/10

(54) **OPHTHALMOLOGIC DEVICE**

(30) Priority: 24.04.2009 JP 2009105753
(71) Applicant: Kabushiki Kaisha TOPCON, Tokyo 174-8580 (JP)
(72) Inventor: NAITO, Tomoko, Tokyo 174-8580 (JP)
(74) Representative: Ilgart, Jean-Christophe
(86) International application number: PCT/JP2010/056909
(87) International publication number: WO 2010/122973

(57) **Abstract**

Provided is an ophthalmologic apparatus capable of measuring a refractive state in a state where a pupil diameter dimension varies with respect to an eye to be examined, the ophthalmologic apparatus including a measurement optical system (30) capable of measuring distribution of an optical characteristic within a pupil diameter of the eye to be examined, the ophthalmologic apparatus comprising visible light illuminating means (80) for illuminating the eye to be examined with visible light along an optical axis of the measurement optical system (30). The visible light illuminating means is configured to be capable of changing an illuminating light intensity in a range including at least from a light intensity capable of exposing the eye to be examined to brightness equivalent to that in a nighttime environment to a light intensity capable of exposing the eye to be examined to brightness equivalent to that in a daytime environment, and the measurement optical system (30) is capable of measuring the distribution of the optical characteristic of the eye to be examined which is illuminated by the visible light illuminating means (80) with the brightness equivalent to that in the nighttime environment and is also capable of measuring the distribution of the optical characteristic of the eye to be examined which is illuminated by the visible light illuminating means (80) with the brightness equivalent to that in the daytime environment.

## Description

### TECHNICAL FIELD

The present invention relates to an ophthalmologic apparatus capable of measuring distribution of an optical characteristic within a pupil diameter of an eye to be examined.

### BACKGROUND ART

Conventionally, there has been known ophthalmologic apparatuses capable of measuring a refractive state of an ophthalmic characteristic of an eye to be examined. There is one of such ophthalmologic apparatuses that includes a measurement optical system capable of measuring distribution of an optical characteristic within a pupil diameter of an eye to be examined. The ophthalmologic apparatus irradiates the ocular fundus of the eye to be examined with a measurement light beam, and performs a wavefront analysis (hereinafter referred to as measurement of wavefront aberration) based on a result of receiving a luminous flux that has passed through the pupil among reflected luminous fluxes of the measurement light beam, thereby being capable of analyzing the refracting power (refractive state) of the eye to be examined from various aspects (see, for example Patent Document 1).

Here, the pupil diameter of the eye to be examined varies between a daytime environment (bright place) and a nighttime environment (dark place). Thus, the refractive state of the pupil diameter in each environment needs to be measured. For this reason, the following is considered. Specifically, the size of the pupil diameter in the daytime (hereinafter referred to as a daytime pupil diameter dimension) is acquired, and, then, the ophthalmologic apparatus is used to perform measurement of the wavefront aberration with respect to the eye to be examined of the pupil being widely dilated in the nighttime, and perform a wavefront analysis thereof to measure the refractive state of the eye to be examined in the nighttime. Also, the wavefront analysis is performed in a region of the daytime pupil diameter dimension, which is concentric with the widely-dilated pupil, to measure the refractive state of the eye to be examined in the daytime.

### PRIOR ART DOCUMENT

### PATENT DOCUMENT

Patent Document 1: JP-A2004-135815

### SUMMARY OF THE INVENTION

### PROBLEMS TO BE SOLVED BY THE INVENTION

However, in an actual eye to be examined, the center position of the pupil may be displaced or the shape of the pupil may be deformed when the pupil is widely dilated or contracted to be small. Accordingly, there is still a room for improving the measurement results of the refractive state of the eye to be examined in daytime environment, which are obtained by the method described in Patent Document 1 mentioned above (made by the same applicant as that of this application).

The present invention has been made in view of the above-described circumstances. Accordingly, an object of the present invention is to provide an ophthalmologic apparatus capable of performing measurement of a refractive state in a state where a pupil diameter dimension thereof varies with respect to an eye to be examined.

### MEANS FOR SOLVING THE PROBLEMS

An embodiment of the invention provides an ophthalmologic apparatus including a measurement optical system capable of measuring distribution of an optical characteristic within a pupil diameter of an eye to be examined, the ophthalmologic apparatus comprising visible light illuminating means for illuminating the eye to be examined with visible light along an optical axis of the measurement optical system, in which the visible light illuminating means is configured to be capable of changing an illuminating light intensity in a range including at least from a light intensity capable of exposing the eye to be examined to brightness equivalent to that in a nighttime environment to a light intensity capable of exposing the eye to be examined to brightness equivalent to that in a daytime environment, and the measurement optical system is capable of measuring the distribution of the optical characteristic of the eye to be examined which is illuminated by the visible light illuminating means with the brightness equivalent to that in the nighttime environment and is also capable of measuring the distribution of the optical characteristic of the eye to be examined which is illuminated by the visible light illuminating means with the brightness equivalent to that in the daytime environment.

Another embodiment of the invention provides the ophthalmologic apparatus wherein the visible light illuminating means is a fixation optical system to project a fixation target image onto the eye to be examined.

Another embodiment of the invention provides the above-described ophthalmologic apparatus wherein the visible light illuminating means is configured to be capable of changing the light intensity between two or more preset values which are intentionally made different, the values including the light intensity capable of exposing the eye to be examined to the brightness equivalent to that in the nighttime environment and the light intensity capable of exposing the eye to be examined to the brightness equivalent to that in the daytime environment.

Another embodiment of the invention provides the above-described ophthalmologic apparatus further comprising: a display unit capable of displaying measurement information on the eye to be examined; an adjustment operation unit to adjust the light intensity of the visible light illuminating means; and pupil diameter dimension measuring means for measuring a pupil diameter dimension of the eye to be examined, wherein the pupil diameter dimension is immediately displayed in the display unit.

Another embodiment of the invention provides the above-described ophthalmologic apparatus further comprising: pupil diameter dimension measuring means for measuring a pupil diameter dimension of the eye to be examined; and a control unit to control the measurement optical system and the visible light illuminating means, in which the control unit changes the light intensity of the visible light illuminating means, and, once the pupil diameter dimension becomes a predetermined size, causes the measurement optical system to execute measurement of the distribution of the optical characteristic of the eye to be examined.

Another embodiment of the invention provides the above-described ophthalmologic apparatus wherein the measurement optical system illuminates an ocular fundus of the eye to be examined with spot light and receives a luminous flux which has passed through the pupil of the eye to be examined after being reflected by the ocular fundus, thereby measuring the distribution of the optical characteristic of the eye to be examined.

Another embodiment of the invention provides the above-described ophthalmologic apparatus wherein the measurement optical system measures a wavefront aberration, thereby measuring the distribution of the optical characteristic of the eye to be examined.

Another embodiment of the invention provides the aove-described ophthalmologic apparatus wherein the optical characteristic of the eye to be examined includes at least a refractive state.

Another embodiment of the invention provides the above-described ophthalmologic apparatus comprising center position measuring means for measuring the center position of the pupil of the eye to be examined.

Another embodiment of the invention provides an ophthalmologic apparatus including a measurement optical system capable of measuring distribution of an optical characteristic within a pupil diameter of an eye to be examined, the ophthalmologic apparatus comprising: visible light illuminating means for illuminating the eye to be examined with visible light along an optical axis of the measurement optical system; and center position measuring means for measuring the center position of the pupil of the eye to be examined, in which the visible light illuminating means is configured to be capable of changing an illuminating light intensity in a range including at least from a light intensity capable of exposing the eye to be examined to brightness equivalent to that in a nighttime environment to a light intensity capable of exposing the eye to be examined to brightness equivalent to that in a daytime environment, and the center position measuring means is capable of measuring the center position of the pupil of the eye to be examined illuminated by the visible light illuminating means with the brightness equivalent to that in the nighttime environment and is also capable of measuring the center position of the pupil of the eye to be examined illuminated by the visible light illuminating means with the brightness equivalent to that in the daytime environment.

### EFFECTS OF THE INVENTION

According to an aspect of an ophthalmologic apparatus of the present invention, an optical characteristic of an eye to be examined in a state where a pupil diameter dimension varies can be measured.

In addition to the above-described configuration, when the visible light illuminating means is the fixation optical system to project a fixation target image onto the eye to be examined, there is no need to provide another light source. Thus, the configuration can be simpler, and a person to be examined only needs to observe the fixation target image during being measured. Accordingly, the pupil diameter can be changed without causing any sense of discomfort of the person to be examined.

In addition to the above-described configuration, in the visible light illuminating means, when the visible light illuminating means is configured to be capable of changing the light intensity between two or more preset values which are intentionally made different, the values including the light intensity capable of exposing the eye to be examined to the brightness equivalent to that in the nighttime environment and the light intensity capable of exposing the eye to be examined to the brightness equivalent to that in the daytime environment, the optical characteristic of the pupil diameter dimension in the daytime environment and the optical characteristic of the pupil diameter dimension in the nighttime environment can be easily measured.

In addition to the above-described embodiment, when the display unit capable of displaying measurement information of the eye to be examined, the adjustment operation unit to adjust a light intensity of the visible light illuminating means, and the pupil diameter dimension measuring means for measuring a pupil diameter dimension of the eye to be examined are included, and the pupil diameter dimension is immediately displayed in the display unit, the optical characteristic in a desired pupil diameter dimension can be measured by operating the adjustment operation unit while visually checking the pupil diameter dimension displayed in the display unit.

In addition to the above-described configuration, when the pupil diameter dimension measuring means for measuring a pupil diameter dimension of the eye to be examined and the control unit to control the measurement optical system and the visible light illuminating means are further included, and the control unit changes the light intensity of the visible light illuminating means, and, once the pupil diameter dimension becomes a predetermined size, causes the measurement optical system to execute measurement of the distribution of the optical characteristic of the eye to be examined, the optical characteristic in a desired pupil diameter dimension can be automatically measured.

In addition to the above-described configuration, when the measurement optical system illuminates the ocular fundus of the eye to be examined with spot light and receives a luminous flux which has passed through the pupil of the eye to be examined after being reflected by the ocular fundus, thereby measuring the distribution of the optical characteristic of the eye to be examined, the optical characteristic containing all the actual optical elements in a region in the eye to be examined which corresponds to the pupil diameter through which the reflected luminous flux is transmitted can be obtained.

In addition to the above-described configuration, when the measurement optical system measures a wavefront aberration, thereby measuring the distribution of the optical characteristic of the eye to be examined, the optical characteristic containing all the actual optical elements in a region in the eye to be examined which corresponds to the pupil diameter through which the reflected luminous flux is transmitted can be obtained.

In addition to the above-described configuration, when the optical characteristic of the eye to be examined includes at least a refractive state, the actual refractive state in a state where a pupil diameter dimension varies can be measured with respect to the eye to be examined.

In addition to the above-described configuration, when center position measuring means for measuring the center position of the pupil of the eye to be examined is included, the center position of the pupil which is displaced along with the pupil being widely dilated or contracted to be small can be properly obtained.

According to the other aspect of an ophthalmologic apparatus of the invention, the ophthalmologic apparatus includes the measurement optical system capable of measuring distribution of an optical characteristic within the pupil diameter of an eye to be examined, the ophthalmologic apparatus comprising: visible light illuminating means for illuminating the eye to be examined with visible light along an optical axis of the measurement optical system; and center position measuring means for measuring the center position of the pupil of the eye to be examined, in which the visible light illuminating means is configured to be capable of changing an illuminating light intensity in a range including at least from a light intensity capable of exposing the eye to be examined to brightness equivalent to that in the nighttime environment to a light intensity capable of exposing the eye to be examined to brightness equivalent to that in the daytime environment, and the center position measuring means is capable of measuring the center position of the pupil of the eye to be examined illuminated by the visible light illuminating means with the brightness equivalent to that in the nighttime environment and is capable of measuring the center position of the pupil of the eye to be examined illuminated by the visible light illuminating means with the brightness equivalent to that in the daytime environment. With this configuration, the center position of the pupil which is displaced along with the pupil being widely dilated or contracted to be small can be properly obtained.

### BRIEF DESCRIPTION OF THE DRAWINGS

FIG 1 is an explanatory diagram schematically showing an optical system of an ophthalmologic apparatus according to the present invention.
FIG 2 is an explanatory diagram schematically showing a block circuit of an electric control system of the ophthalmologic apparatus.
FIG 3 is an explanatory diagram schematically showing the front view of a Placido ring pattern which is seen from the eye to be examined side.
FIG 4 is an explanatory diagram for illustrating a projected image by the Placido ring pattern seen on an area sensor (a light-receiving surface thereof).
FIG 5 is an explanatory diagram similar to that of FIG 4 for illustrating a state where the Z-alignment is displaced.
FIG 6A is an explanatory diagram for illustrating how the pupil of the eye to be examined changes and shows a state where the pupil is widely dilated.
FIG 6B is an explanatory diagram for illustrating how the pupil of the eye to be examined changes and shows a state where the pupil is contracted and an estimated state thereof.
FIG 7 is an explanatory diagram for illustrating how a wavefront aberration is measured with regard to the eye to be examined with the pupil thereof being contracted.
FIG 8 is an explanatory diagram for illustrating how a wavefront aberration is measured with regard to the eye to be examined with the pupil thereof being widely dilated.
FIG 9 is an explanatory diagram similar to that of FIG 2 showing an electronic control system of an ophthalmologic apparatus according to Modification 1.
FIG 10 is an explanatory diagram showing an example in which a pupil diameter dimension is displayed in real time in a display unit in the ophthalmologic apparatus according to Modification 1.
FIG 11 is an explanatory diagram showing another example in which a pupil diameter dimension is displayed in real time in a display unit in the ophthalmologic apparatus according to Modification 1.
FIG 12 is an explanatory diagram similar to that of FIG 2 showing an electronic control system of an ophthalmologic apparatus according to Modification 2.

### MODES FOR CARRYING OUT THE INVENTION

Hereinafter, embodiments of an ophthalmologic apparatus according to the present invention are described by referring to the drawings.

### Embodiment 1

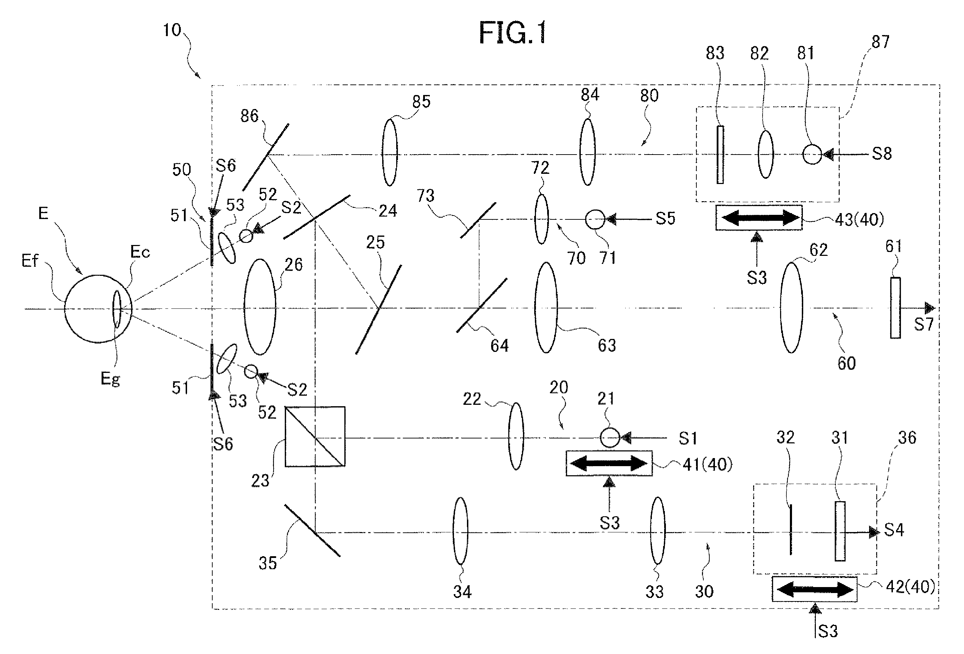

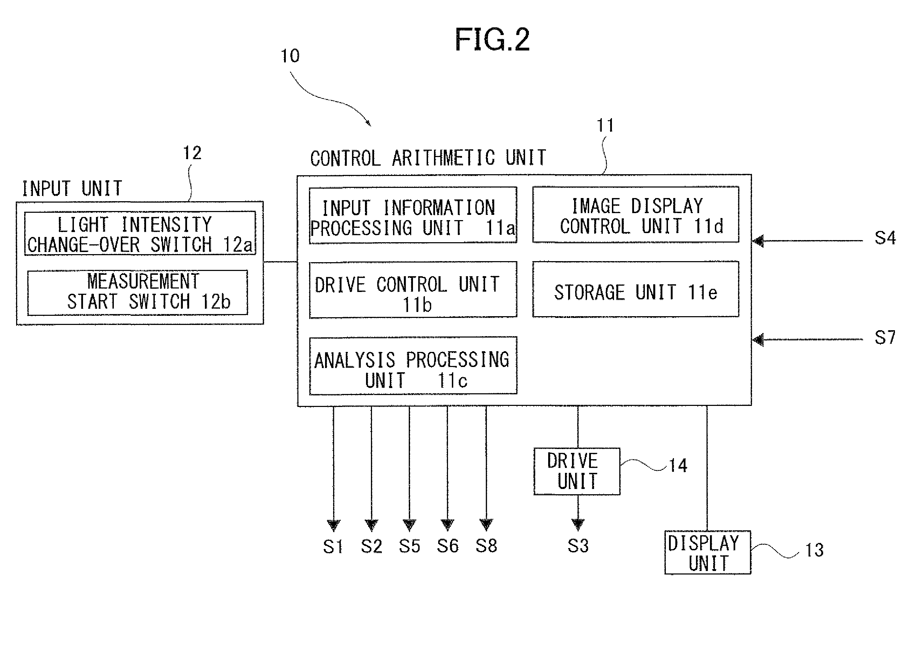

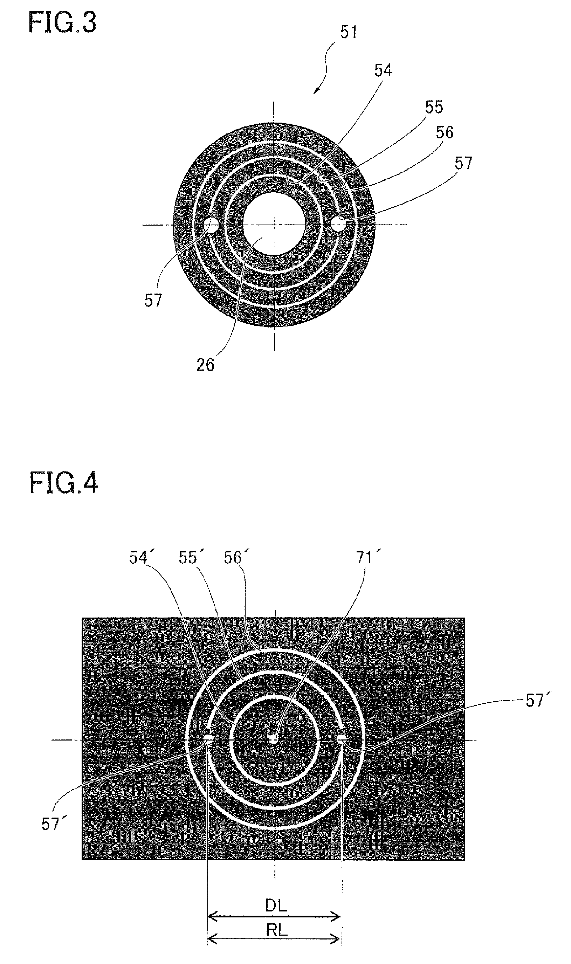

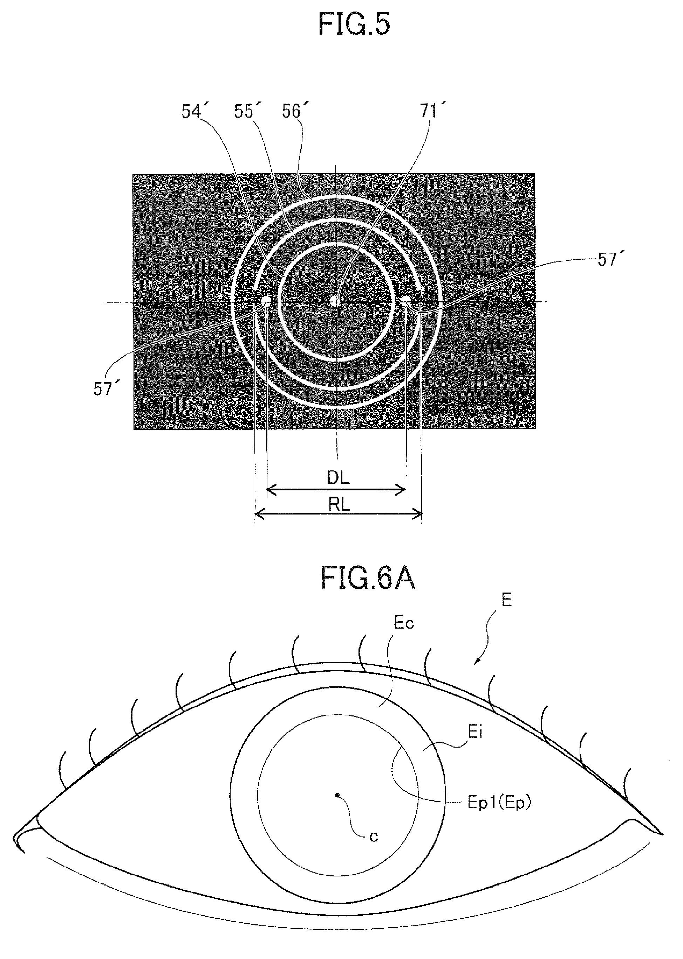

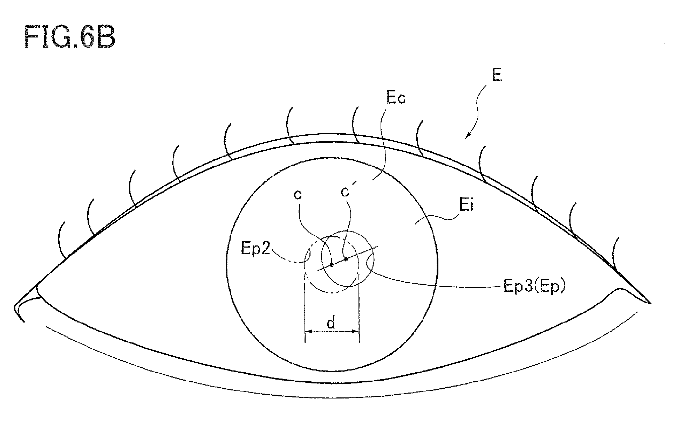

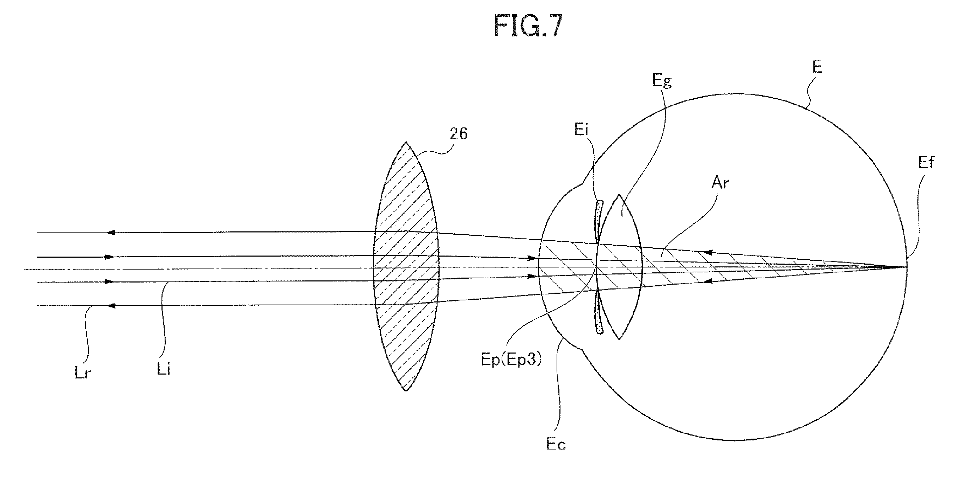

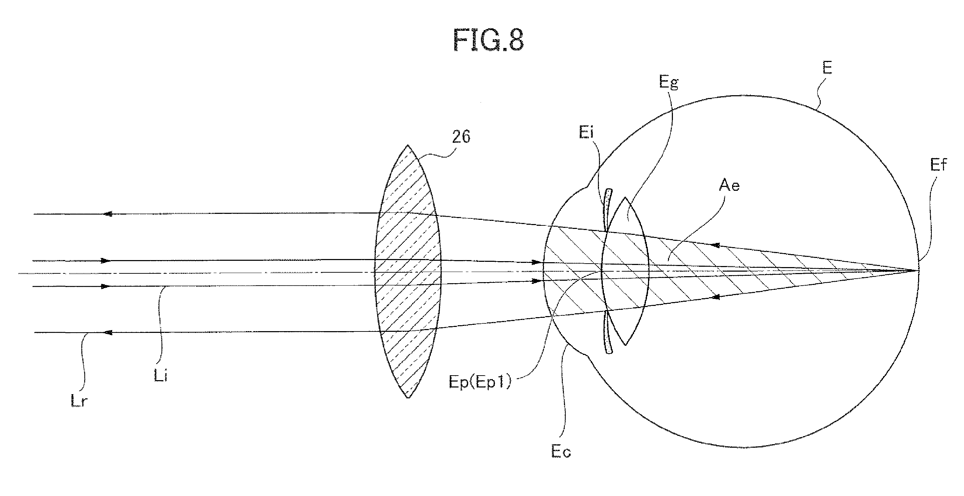

FIG 1 is an explanatory diagram schematically showing an optical system of an ophthalmologic apparatus 10 according to the invention. FIG 2 is an explanatory diagram schematically showing a block circuit of an electric control system of the ophthalmologic apparatus 10. FIG 3 is an explanatory diagram schematically showing the front view of a Placido ring pattern 51 which is seen from the eye E to be examined side. FIG 4 is an explanatory diagram for illustrating a projected image by the Placido ring pattern 51, which is seen on an area sensor 61 (a light-receiving surface thereof). FIG 5 is an explanatory diagram similar to that of FIG 4 for illustrating a state where the Z-alignment is displaced. FIG 6A and FIG 6B are explanatory diagrams, each illustrating how the pupil Ep of the eye E to be examined changes. FIG 6A shows a state where the pupil Ep is widely dilated, while FIG 6B shows a state where the pupil Ep is contracted and an estimated state thereof. FIG 7 is an explanatory diagram for illustrating how a wavefront aberration is measured with respect to the eye E to be examined with the pupil Ep thereof being contracted. FIG 8 is an explanatory diagram for illustrating how a wavefront aberration is measured with respect to the eye E to be examined with the pupil Ep thereof being widely dilated. Note that for easy understanding, the displacement of the center position and the change in the shape of the pupil when the pupil is contracted are emphasized in FIG 6B, which does not comply with the actual aspect of the change in the eye E to be examined.

In Embodiment 1, the ophthalmologic apparatus 10 is a wavefront aberration measuring device to measure a wavefront aberration so as to measure an optical characteristic of the eye E to be examined. For this eye E to be examined, FIG 1 shows a retina (ocular fundus) Ef, a cornea (anterior eye part) Ec, and a crystalline lens Eg.

As shown in FIG 1, the ophthalmologic apparatus 10 includes as optical systems a measurement illumination system 20, a light-receiving optical system 30, optical system moving means 40, an anterior eye part illumination system 50, an alignment observation optical system 60, an XY-alignment optical system 70, and a fixation optical system 80. In addition, as shown in FIG 2, the ophthalmologic apparatus 10 includes as electric control systems, a control arithmetic unit 11, an input unit 12, a display unit 13, and a drive unit 14.

Hereinafter, the description is given to the brief configuration of the optical systems of the ophthalmologic apparatus 10 according to the invention to which a model eye is applied, the brief configuration of the block circuit diagram, and effects of this ophthalmologic apparatus.

### (Configuration of the measurement illumination system 20)

As shown in FIG 1, the measurement illumination system 20 is used to emit spot light as an illumination luminous flux to the ocular fundus Ef of the eye E to be examined (see, FIG 7 and FIG 8). This measurement illumination system 20 includes a measurement light source 21, a lens 22, a polarization beam splitter 23, a dichroic mirror 24, a dichroic mirror 25, and an objective lens 26. The measurement illumination system 20 has the polarization beam splitter 23 and the dichroic mirrors 24, 25 which are disposed between the lens 22 and the objective lens 26. The polarization beam splitter 23 is configured of a dichroic mirror which reflects a P-polarization component of illumination luminous flux and transmits an S-polarization component of the reflected luminous flux from an ocular fundus Ef to be described later. The dichroic mirror 24 is configured of a wavelength selective mirror which reflects the illumination luminous flux and the reflected luminous flux and transmits a fixation luminous flux to be described later. The dichroic mirror 25 is configured of a dichroic mirror which reflects the illumination luminous flux, the reflected luminous flux, and the fixation luminous flux and transmits an observation luminous flux to be described later.

Note that the optical elements from the eye E to be examined to the dichroic mirror 25 are commonly used among the measurement illumination system 20, the light-receiving optical system 30, the alignment observation optical system 60, the XY-alignment optical system 70, and the fixation optical system 80. The optical elements from the dichroic mirror 25 to the dichroic mirror 24 are commonly used among the measurement illumination system 20, the light-receiving optical system 30, and the fixation optical system 80. The optical elements from the dichroic mirror 24 to the polarization beam splitter 23 are commonly used among the measurement illumination system 20 and the light-receiving optical system 30.

In Embodiment 1, the measurement light source 21 uses an SLD (super-luminescent diode) emitting near infrared ray. Note that the measurement light source 21 may use a laser, an LED, or the like. This measurement light source 21 is controlled by a control signal S1 from the control arithmetic unit 11 (see FIG 2).

This measurement illumination system 20 reflects the luminous flux outputted from the measurement light source 21 and transmitted through the lens 22 with the polarization beam splitter 23, the dichroic mirror 24, and the dichroic mirror 25 to guide the luminous flux onto the optical axis of the objective lens 26, thereby illuminating the retina (ocular fundus) Ef of the eye E to be examined through the objective lens 26.

### (Configuration of the light-receiving optical system 30)

The light-receiving optical system 30 serves to guide the reflected luminous flux from the ocular fundus Ef, which is caused by the illumination of the measurement illumination system 20, to an area sensor 31. This light-receiving optical system 30 has the area sensor 31, a Hartmann plate 32, a lens 33, a lens 34, and a reflecting mirror 35. In addition, as described above, in the light-receiving optical system 30, the optical systems from the eye E to be examined to the polarization beam splitter 23 are common with the optical systems of the measurement illumination system 20. The reflecting mirror 35 serves to cause the optical axis of the reflected luminous flux from the ocular fundus Ef to be parallel with the direction of the optical axis of the illumination luminous flux which is outputted from the measurement light source 21. In other words, in the light-receiving optical system 30, the reflected luminous flux from the retina (ocular fundus) Ef of the eye E to be examined, which is illuminated by the measurement illumination system 20, passes through the objective lens 26, and then is reflected by the dichroic mirror 24 and the dichroic mirror 25, and is caused to transmit through the polarization beam splitter 23 to be reflected by the reflecting mirror 35, thereby being guided onto the measurement optical axis on which the lens 33, the lens 34, and the Hartmann plate 32 are disposed. The lens 33 guides the reflected luminous flux having passed through the lens 34 to the Hartmann plate 32 after being converted to the parallel luminous flux. The Hartmann plate 32 has a function to divide the luminous flux into multiple divided luminous fluxes. The reflected luminous flux from the ocular fundus Ef is divided by the Hartmann plate 32 into multiple divided luminous fluxes, and each of the divided luminous fluxes is collected on the light-receiving surface of the area sensor 31. The area sensor 31 receives the multiple divided luminous fluxes and performs the photoelectric conversion thereon, so that the area sensor 31 receives the multiple divided luminous fluxes divided by the Hartmann plate 32 and outputs a light-receiving signal S4 corresponding to the received light intensity of each divided luminous flux. A wavefront aberration is obtained based on the light-receiving signal S4 from this area sensor 31. By performing an analysis (performing the Zernike analysis based on a gradient angle of the luminous flux obtained by the area sensor 31) based on the change in this wavefront aberration (a movement amount with respect to an ideal wavefront of each bright point on the light-receiving surface of the area sensor 31), the optical characteristic (a refractive state, amount of aberration, or the like) of the eye E to be examined can be calculated. For this reason, the light-receiving optical system 30 functions as a measurement optical system capable of measuring the optical identity distribution within the pupil diameter of the eye E to be examined. The light-receiving signal S4 is outputted to the control arithmetic unit 11 (see FIG 2). This area sensor 31 uses an area CCD in Embodiment 1. Note that the area sensor 31 may use a CMOS sensor or the like.

The Hartmann plate 32 which is used in the light-receiving optical system 30 is a member to divide the luminous flux into multiple divided luminous fluxes. Embodiment 1 uses multiple microlenses, such as micro Fresnel lenses, which are disposed within a surface perpendicular to the optical axis.

### (Configuration of the optical system moving means 40)

The optical system moving means 40 has light source moving means 41 for moving the measurement light source 21, sensor moving means 42 for moving the sensor unit 36, and target moving means 43 for moving a target unit 87 to be described later. The light source moving means 41 has a function to move the measurement light source 21 along the measurement optical axis of the measurement illumination system 20. The sensor moving means 42 has a function to move the sensor unit 36 along the measurement optical axis of the light-receiving optical system 30. The target moving means 43 is described later.

Each of the light source moving means 41 and the sensor moving means 42 is driven depending on a refractivity of the eye E to be examined so that the measurement light source 21, the retina (ocular fundus) Ef of the eye E to be examined, and the area sensor 31 (the light-receiving surface thereof) would have a substantially-conjugated positional relationship. As the foregoing moving method, it is only needed that a distance between the bright points on the area sensor 31 when the eye E to be examined with "0" diopter (hereinafter described as "0"D) is measured is stored in advance and the distance between the bright points on the area sensor 31 when the eye E to be examined is actually measured is moved to a position substantially matching with the stored distance. In Embodiment 1, the measurement illumination system 20 and the light-receiving optical system 30 are optically configured so that the movement amount of the measurement light source 21 and the movement amount of the sensor unit 36 would be equal to each other. The measurement light source 21 and the sensor unit 36 are linked. The light source moving means 41 and the sensor moving means 42 are configured as single optical system moving means 40 which is driven by a single driving source (for example, a motor). Also, as described later, the target moving means 43 is configured as the single optical system moving means 40. This optical system moving means 40 is driven and controlled by a movement control signal S3 from the drive unit 14 (see FIG 2).

### (Configuration of the anterior eye part illumination system 50)

The anterior eye part illumination system 50 serves to illuminate the anterior eye part (cornea) Ec with a illumination light having a predetermined pattern, and is used for measuring a curvature of the cornea Ec of the eye E to be examined and for Z-alignment to keep a constant distance between the eye E to be examined and the apparatus. The anterior eye part illumination system 50 has a Placido ring pattern 51, a light source LED 52, and a collimator lens 53. As shown in FIG 3, the Placido ring pattern 51 has multiplexed ring patterns 54, 55, 56 which transmit light and a pair of apertures 57. The ring patterns 54, 55, 56 are adapted to concentrically surround the objective lens 26 (using the optical axis of the objective lens 26 as the center thereof). The pair of apertures 57 are provided on the straight line passing through the center of the ring pattern 55 (perpendicular to the optical axis of the objective lens 26), and on the ring pattern 55, a diameter dimension of the ring pattern 55 and an distance between the center positions of the apertures 57 are set to be equal. The Placido ring pattern 51 is illuminated by an LED (not shown) disposed on the back surface and illuminates the cornea Ec of the eye E to be examined with a ring-shaped light-emitting pattern by the luminous flux having transmitted through the ring patterns 54, 55, 56. The LED provided on the back surface of the Placid ring pattern 51 is controlled by a control signal S6 from the control arithmetic unit 11 (see FIG 2).

The light source LED 52 is provided on the back surface side of the Placid ring pattern 51 (the objective lens 26 side when seen from the eye E to be examined) corresponding to each of the apertures 57. The collimator 53 is provided between the light source LED 52 and the Placido ring pattern 51 so that a focal position would be an outgoing position of the light source LED 52. The luminous flux which is outputted from the light source LED 52 towards the back surface of the Placido ring pattern 51 is set to be parallel luminous fluxes. Each of the parallel luminous fluxes passes through the corresponding aperture 57 to illuminate the cornea Ec of the eye E to be examined. This light source LED 52 is controlled by a control signal S2 from the control arithmetic unit 11 (see FIG 2).

This anterior eye part illumination system 50 illuminates the cornea Ec by the Placid ring pattern 51 as a ring-shaped light-emitting pattern having passed through the ring patterns 54, 55, 56 and illuminates the cornea Ec with the parallel luminous fluxes from the light source LED 52. These luminous fluxes are reflected by the cornea Ec (the surface thereof) and the reflected luminous fluxes (hereinafter referred to as observation luminous fluxes) transmit through the objective lens 26 and the dichroic mirror 25, and pass through the alignment observation optical system 60 to be described later, and then form on the area sensor 61 ring-shaped projected images 54', 55', 56' made by the ring patterns 54, 55, 56 of the Placido ring pattern 51 as well as a pair of bright point images 57' which are made by each light source LED 52 and have passed through the pair of apertures 57 (see, FIG 4 and FIG 5). Note that in Embodiment 1, in addition to the ring-shaped projected images 54', 55', 56' and the pair of bright point images 57', a bright point image 71' for XY-alignment to be described later is formed on the area sensor 61 (see, FIG 5 and FIG 5). The ring diameters of the projected images 54', 55', 56' which are formed on this area sensor 61 depend on the curvature of the cornea Ec of the eye E to be examined. Accordingly, the curvature of the cornea Ec can be measured based on the light-receiving signal (the ring diameter on the area sensor 61) from the area sensor 61. In addition, when the number of the rings (the ring patterns 54, 55, 56) in the Placid ring pattern 51 is increased or the distance between the rings is caused to be closer, not only the curvature of the cornea Ec but also the detailed shape of the cornea Ec can be measured.

Here, the ring diameters of the projected images 54', 55', 56' which are formed on the area sensor 61 are affected by the operating distance of the apparatus body with respect to the eye E to be examined (an optical distance from the apex of the cornea to the objective lens 26), in other words, the positions of the measurement illumination system 20, the light-receiving optical system 30, and the alignment observation optical system 60 with respect to the eye E to be examined in the optical axis direction. Accordingly, the distance (positional relationship) has to be kept constant, which is referred to as Z-alignment. This Z-alignment is needed not only for measuring the shape of the cornea Ec but also for measuring the wavefront aberration. The Z-alignment is performed as follows.

As described above, the anterior eye part illumination system 50 illuminates the cornea Ec as the bright point by the pair of light sources LED 52 in addition to the ring-shaped light-emitting pattern. This luminous flux is reflected by the cornea Ec (the surface thereof), and then this reflected observation luminous flux is transmitted through the objective lens 26 and the dichroic mirror 25 and passes through the alignment observation optical system 60 to be described later before forming on the area sensor 61 a pair of the bright point images 57' by the pair of the light sources LED 52 in addition to the ring-shaped projected images 54', 55', 56' (see FIG 4). As described above, in the Placido ring pattern 51, the pair of the apertures 57 are provided on the ring pattern 55, and the diameter dimension of the ring pattern 55 and an distance between the center positions of the both apertures 57 are set to be equal. In addition, in the anterior eye part illumination system 50, the illuminating direction with respect to the cornea Ec is inclined to the optical axis direction of the objective lens 26. Also, the outgoing position of the LED provided on the back surface of the Placido ring pattern 51 when seen from the optical axis direction and the outgoing position of the light source LED 52 are set to be different. For this reason, when seen by the images formed by the anterior eye part illumination system 50 on the area sensor 61 (the light-receiving surface thereof), if the Z-alignments are equal to each other, the diameter dimension RL of the ring-shaped projected image 55' and an distance DL between the center positions of the pair of the bright point images 57' becomes equal to each other (see, FIG 4). In addition, when seen by the images formed by the anterior eye part illumination system 50 on the area sensor 61 (the light-receiving surface thereof), the diameter dimension RL of the ring-shaped projected image 55' changes according to the change in the Z-alignment, although the distance DL between the center positions of the pair of the bright point images 57' does not change regardless of the change in the Z-alignment. For this reason, if the Z-alignments are not equal on the area sensor 61 (the light-receiving surface thereof), there causes a difference between the diameter dimension RL of the ring-shaped projected image 55' and the distance DL between the center positions of the pair of the bright point images 57' (see FIG 5). Accordingly, the position of the apparatus is moved forward or backward with respect to the eye E to be examined so that the diameter dimension RL of the ring-shaped projected image 55' and the distance DL between the center positions of the pair of the bright point image 57' would be equal to each other. Consequently, the Z-alignment to keep the distance between the eye E to be examined and the device constant can be executed.

### (Configuration of the alignment observation optical system 60)

The alignment observation optical system 60 serves to observe the anterior eye part Ec by using the observation luminous flux which is the illumination luminous flux illuminated from the anterior eye part illumination system 50 and is reflected in the anterior eye part Ec of the eye E to be examined. This alignment observation optical system 60 has the area sensor 61, a lens 62, a lens 63, and a half mirror 64. In addition, as described above, it is adapted in the alignment observation optical system 60 that optical systems from the eye E to be examined to the dichroic mirror 25 are common with the optical systems of the measurement illumination system 20. The half mirror 64 is disposed between the dichroic mirror 25 and the lens 63 to transmit the observation luminous flux reflected in the cornea Ec towards the area sensor 61 and reflect an adjustment luminous flux outputted from an XY alignment optical system 70 to be described later towards the objective lens 26. The lens 62 converts the reflected luminous flux into collected luminous flux to guide the luminous flux to the area sensor 61. The area sensor 61 is configured of, for example, a CCD. As described above, the ring-shaped projected images 54', 55', 56' by the anterior eye part illumination system 50, the pair of the bright point images 57', and a bright point image 71' for XY-alignment by an XY-alignment optical system 70 to be described later are formed on the light-receiving surface of the area sensor 61 (CCD). This area sensor 61 sends a light-receiving signal S7 to the control arithmetic unit 11 (see, FIG 2).

### (Configuration of the XY-alignment optical system 70)

The XY-alignment optical system 70 serves to perform alignment adjustment on the eye E to be examined in the XY direction (within a face vertical to the optical axis, such as the measurement illumination system 20 and the light-receiving optical system 30 in the vicinity of the eye E to be examined). The XY-alignment optical system 70 has an alignment light source 71, a lens 72, and a reflecting mirror 73. In addition, as described above, in the XY-alignment optical system 70, optical systems from the eye E to be examined to the half mirror 64 are common with the optical systems of the alignment observation optical system 60. The alignment light source 71 is controlled by a control signal S5 from the control arithmetic unit 11 (see FIG 2). In the XY-alignment optical system 70, the luminous flux which is outputted from the alignment light source 71, passes through the lens 72, and then is reflected by the reflecting mirror 73, is caused to be reflected by the half mirror 64, so as to pass through the dichroic mirror 25 and the objective lens 26 to illuminate the cornea Ec of the eye E to be examined. Here, the area sensor 61 is disposed so as to be substantially conjugated with a virtual image (Purkinje image) made by the curvature of the cornea Ec. The cornea Ec is illuminated from the XY-alignment optical system 70, so that the luminous flux (hereinafter referred to as an adjustment luminous flux) reflected by the cornea Ec (the surface thereof) is transmitted through the objective lens 26 and the dichroic mirror 25, passes through the alignment observation optical system 60, and then forms the bright point image 71' for XY-alignment on the area sensor 61. As shown in FIG 4, in this XY-alignment optical system 70, when the apex of the cornea of the eye E to be examined matches with the optical axis of the alignment observation optical system 60, it is set such that the bright point image 71' for XY-alignment would be positioned in the center on the area sensor 61 (the light-receiving surface thereof). Here, when the apex of the cornea of the eye E to be examined moves within a plane surface perpendicular to the optical axis of the alignment observation optical system 60, the bright point image 71' for XY-alignment moves on the area sensor 61 (the light-receiving surface thereof) depending on the movement amount thereof. For this reason, when the apparatus body is moved with respect to the eye E to be examined so that the bright point image 71' for XY-alignment would be positioned in the center on the area sensor 61 (the light-receiving surface thereof), the XY-alignment can be executed.

### (Configuration of the fixation optical system 80)

The fixation optical system 80 serves to project a target to an eye E to be examined for fixation or fogging, for example. The fixation optical system 80 is an optical system to project a fixation target image to the eye E to be examined and has a light source 81, a lens 82, a fixation target 83, a lens 84, a lens 85, and a reflecting mirror 86. In addition, as described above, in the fixation optical system 80, optical systems from the eye E to be examined to the dichroic mirror 24 are common with the optical systems of the measurement illumination system 20. The reflecting mirror 86 serves to cause a luminous flux which is outputted from the light source 81 and is transmitted through the lens 82, the fixation target 83, the lens 84, and the lens 85 (hereinafter referred to as a fixation luminous flux) to be aligned with the direction of the optical axis of the illumination luminous flux from the measurement light source 21 in the measurement illumination system 20 and the direction of the optical axis of the reflected luminous flux towards the area sensor 31 in the light-receiving optical system 30. The light source 81 is a light source outputting light with a wavelength in a visible region (hereinafter simply referred to as visible light) and uses a tungsten lamp or LED. Also, the light source 81 is set so as to have a variable light intensity. It is assumed that this variable range includes a range at least from a light intensity capable of exposing the eye E to be examined, the eye E being caused to observe the fixation target image, to brightness equivalent to that in the nighttime environment to a light intensity capable of exposing the eye E to be examined to brightness equivalent to that in the daytime environment. For this reason, the fixation optical system 80 (the light source 81 thereof) functions as visible light illuminating means for illuminating the eye E to be examined with visible light along the optical axis of the measurement optical system. In this Embodiment 1, the light source 81 is set so that the brightness can be switched over in four levels, from a light intensity capable of emitting brightness equivalent to that in the nighttime environment to a light intensity capable of emitting brightness equivalent to that in the daytime environment, and is controlled by a control signal S8 from the control arithmetic unit 11 (see FIG 2) according to the operation of the input unit 12 over the light intensity change-over switch 12a.

Although an illustration is omitted, the fixation target 83 includes a pattern of landscapes or radiation ray, and is illuminated from the back side with the luminous flux outputted from the light source 81. In this fixation optical system 80, the visible light which is outputted from the light source 81 and is transmitted through the fixation target 83 (hereinafter referred to as a fixation luminous flux) is caused to transmit through the lens 84 and the lens 85, be reflected by the reflecting mirror 86, transmit through the dichroic mirror 24, be reflected by the dichroic mirror 25, pass through the objective lens 26, and enter into the eye E to be examined. In this manner, the fixation target 83 is projected on the retina (ocular fundus) Ef and the eye E to be examined is caused to observe the fixation target image. With this, the line of sight of the eye E to be examined can be fixed on the fixation target 83.

The target unit 87 in this fixation optical system 80, which includes the light source 81, the lens 82, and the fixation target 83, is set so as to be movable along the fixation optical axis of the fixation optical system 80 by the target moving means 43. The target moving means 43 is driven so as to move the fixation optical system 80 up to a position where an image of the fixation target 83 can be formed (a position where an image is brought into focus) on the retina (ocular fundus) Ef. In addition, in the case where a degree of the eye E to be examined is measured, the target moving means 43 performs fogging to move up to the position where an image is out of focus in order to eliminate effects of the adjustment of the eye E to be examined. Note that in Embodiment 1, the measurement light source 21 and the sensor unit 36 are linked with each other and the target moving means 43, as well as the sensor moving means 42 and the light source moving means 41, is configured as single optical system moving means 40, and is driven by the single driving source (for example, a motor). For this reason, the target unit 87 is driven and controlled by the moving control signal S3 sent to the target moving means 43, or the optical system moving means 40.

### (Configuration of an electric circuit)

As described above, the ophthalmologic apparatus 10 includes the control arithmetic unit 11, the input unit 12, the display unit 13, and the drive unit 14 as an electric control system as shown in FIG 2.

The light-receiving signal S4 from the area sensor 31 of the light-receiving optical system 30 and the light-receiving signal S7 from the area sensor 61 of the alignment observation optical system 60 are input to the control arithmetic unit 11. Also, an operation signal from the input unit 12 is input to the control arithmetic unit 11. This control arithmetic unit 11 has an input information processing unit 11a, a drive control unit 11b, an analysis processing unit 11c, a image display control unit 11d, and a storage unit 11e.

The input information processing unit 11a processes the light-receiving signal S4 and light-receiving signal S7 to be input and the operation signal from the input unit 12 as needed and sends them to the drive control unit 11b, the analysis processing unit 11c, the image display control unit 11d, and the storage unit 11e.

Based on the signal from the input information processing unit 11a (such as the operation signal from the input unit 12), the drive control unit 11b drives and controls (turns on or turns out) the measurement light source 21 of the measurement illumination system 20, drives and controls the optical system moving means 40, and drives and controls the Placido ring pattern 51 and the light source LED 52 of the anterior eye part illumination system 50, the alignment light source 71 of the XY-alignment optical system 70, the light source 81 of the fixation optical system 80, and the drive unit 14. Also, the drive control unit 11b performs control based on the signal corresponding to the calculation result of the analysis processing unit 11c. In other words, the control signals S1, S2, S5, S6, S8 are sent to control the measurement light source 21 of the measurement illumination system 20, the Placido ring pattern 51 and the light source LED 52 of the anterior eye part illumination system 50, the alignment light source 71 of the XY-alignment optical system 70, the light source 81 of the fixation optical system 80, or the moving control signal S3 is sent to the optical system moving means 40 by driving the drive unit 14. Furthermore, the drive control unit 11b performs various kinds of controls to fulfill the functions of the ophthalmologic apparatus 10. After that, the automatic control may be performed for adjusting the light intensity of the light outputted from the measurement light source 21 using control programs stored in the storage unit 11e.

The analysis processing unit 11c calculates the wavefront aberration, refracting power, and the like of the eye E to be examined based on the signals from the input information processing unit 11a (the light-receiving signal S4 from the light -receiving optical system 30 and the light-receiving signal S7 from the alignment observation optical system 60). Also, the analysis processing unit 11c calculates various optical characteristics relating to the eye E to be examined: for example, point spread function (PSF), MTF (Modulation Transfer Function) indicating a transfer characteristic of the eye to be examined, a pupil diameter dimension, a contrast sensitivity, and the like, from the measured wavefront aberration and other measured data. For this reason, the analysis processing unit 11c functions as pupil diameter dimension measuring means. Furthermore, the analytical processing unit 11c outputs, as needed, a signal or other signal data corresponding to the calculation result to the drive control unit 11b which performs control of the optical systems and the electric control system and the image display control unit 11d, and the storage unit 11e.

The image display control unit 11d outputs a signal to cause an image of an anterior eye part Ec of the eye E to be examined or an image showing a wavefront thereof to be displayed based on a signal from the input information processing unit 11a (such as a light-receiving signal S4 from the light-receiving optical system 30 and a light-receiving signal S7 from the alignment observation optical system 60). In addition, the image display control unit 11d outputs a signal to the display unit 13 to cause measurement results, calculation results, analysis results, a window to which an operator inputs data or gives an instruction, or the like to be displayed.

The storage unit 11e stores data relating to the eye E to be examined, the data to be used for calculation of the wavefront aberration, the set data for measurement, and the like. In other words, the information sent from the input information processing unit 11a, the drive control unit 11b, and the analysis processing unit 11c are stored as needed, and the stored information is taken out as needed in response to a request from the input information processing unit 11a, the drive control unit 11b, and the analytical processing unit 11c, or the image display control unit 11d. Also, the storage unit 11e stores control programs which are used when the measurement is automatically performed.

The input unit 12 is a switch, button, keyboard, or the like for an operator to input various kinds of input signals, such as predetermined setting, instructions, data, and the like. Here, it is assumed that the input unit 12 includes a pointing device or the like for supporting the button, icon, position, region, or the like which is shown on the display unit 13. The input unit 12 outputs an operation signal corresponding to the operation made therein to the control arithmetic unit 11. In Embodiment 1, the input unit 12 has a light intensity change-over switch 12a and a measurement start switch 12b. The light intensity change-over switch 12a is for switching brightness so as to have set four levels of brightness, from a light intensity capable of emitting brightness equivalent to that in the nighttime environment to a light intensity capable of emitting brightness equivalent to that in the daytime environment, and the measurement start switch 12b is for executing various kinds of measurements.

The display unit 13 displays measurement results, calculation results, analysis results, a window to which an operator inputs data, an image of the eye E to be examined, and the like. The display unit 13 performs displays as needed under the control of the control arithmetic unit 11.

For example, based on the light-receiving signal S4 from the area sensor 31, which is input to the control arithmetic unit 11, the drive unit 14 drives the optical system moving means 40 which integrally moves the measurement light source 21 (light source moving means 41) of the measurement illumination system 20, the sensor unit 36 (sensor moving unit 42) of the light-receiving optical system 30, and the target unit 87 (the target moving means 43) of the fixation optical system 80 in the optical axis direction. This drive unit 14 drives the optical system moving means 40 by outputting the measurement control signal S3 to the optical system moving means 40.

### (Outline of the measurement of wavefront aberration)

When the measurement of the wavefront aberration is performed in this ophthalmologic apparatus 10, as shown in FIG 1, the light source 81 of the fixation optical system 80 is turned on and the eye E to be examined is caused to observe the fixation target image. In this state, the XY-alignment allows the apex of the cornea of the eye E to be examined and the measurement optical axis of the apparatus body (the optical axis of the objective lens 26) to be aligned with each other, and the Z-alignment allows the distance from the apex of the cornea of the eye E to be examined to the apparatus body to be kept constant. Thereafter, the measurement light source 20 of the measurement illumination system 20 is moved by the optical system moving means 40 to a reference position to turn on the measurement light source 21. At this time, the sensor unit 36 of the light-receiving optical system 30 and the target unit 87 of the fixation optical system 80 are also integrally moved by the optical system moving means 40, thereby being moved to the reference position. In this reference position, the refractive state of the eye E to be examined is temporarily measured. Based on the temporal measurement result, the measurement light source 21 of the measurement illumination system 20, the sensor unit 36 of the light-receiving optical system 30, and the target unit 87 of the fixation optical system 80 are moved to a position where the refracting power of the eye E to be examined is negated, and then the refractive state of the eye E to be examined is measured again in that position. As a result of the measurement performed again, if the sensor unit 36 of the light-receiving optical system 30 is in the position where the refracting power of the eye E to be examined is substantially negated, the target unit 87 of the fixation optical system 80 is moved to a plus side to cause the fixation target image to be fogged. In this state, the refractive state and aberration of the eye E to be examined are measured.

In this measurement, in the measurement illumination system 20, the luminous flux which is outputted from the measurement light source 21 and is transmitted through the lens 22 is reflected by the polarization beam splitter 23, the dichroic mirror 24, and the dichroic mirror 25 and is guided onto the optical axis of the objective lens 26, so as to illuminate the ocular fundus Ef of the eye E to be examined after passing through the objective lens 26. If this is set as an illumination luminous flux Li, as shown in FIG 7 and FIG 8, the luminous flux enters into the eye E to be examined as a luminous flux with an extremely small diameter after passing through the objective lens 26 and illuminates a minute region (spot light) of the ocular fundus Ef. Then, the illumination luminous flux Li is reflected in the ocular fundus Ef, and the luminous flux of the reflected luminous flux, which passes through the pupil Ep (inside of an Iris Ei), goes to the objective lens 26. If this is set as a reflected luminous flux Lr, the reflected luminous flux Lr is, as shown in FIG 1, guided to the light-receiving optical system 30 after passing through the objective lens 26. In other words, the reflected luminous flux Lr is reflected by the dichroic mirror 24 and the dichroic mirror 25 after the objective lens 26 in the light-receiving optical system 30, is transmitted through the polarization beam splitter 23, is reflected by the reflecting mirror 35, goes to the lens 33, lens 34, the Hartmann plate 32, and, after the Hartmann plate 32, is focused on the light-receiving surface of the area sensor 31 after being divided into multiple divided luminous fluxes. By receiving each of the divided luminous fluxes and then performing photoelectric conversion on the received divided luminous flux, the area sensor 31 outputs the light-receiving signal S4 corresponding to the received light intensity of each divided luminous flux to the control arithmetic unit 11. The control arithmetic unit 11 can obtain the wavefront aberration from the data acquired by the light-receiving signal S4 in the analysis processing unit 11c. The control arithmetic unit 11 performs analysis based on the change in the wavefront aberration (the movement amount of each bright point on the light-receiving surface of the area sensor 31 with respect to an ideal wavefront), so that the optical characteristic of the eye E to be examined (such as a refractive state or aberration amount) can be calculated. Here, as shown in FIG 7 and FIG 8, this calculated refractive state becomes one containing all of the actual optical elements in the region in the eye E to be examined where the reflected luminous flux Lr is transmitted (see, the region shown by hatching in FIG 7 (reference sign Ar) and the region shown by hatching in FIG 8 (reference sign Ae)).

### (Problems of the conventional art)

Since a pupil diameter of an eye to be examined changes according to the daytime environment (bright place)(hereinafter simply referred to as "daytime") or the nighttime environment (dark place)(hereinafter simply referred to as "nighttime"), a refractive state of each pupil diameter needs to be measured in order to obtain a more correct refractive state under each of the environments. Here, as described above, the measurement of wavefront aberration can obtain the refraction sate that contains all of the actual optical elements in a region in an eye E to be examined where a reflected luminous flux Lr is transmitted (see reference sign Ar in FIG 7 and reference sign Ae in FIG 8). Accordingly, the refractive states of the eye to be examined in the daytime and the nighttime are conventionally measured as described below.

Firstly, as shown in FIG 8, an illumination luminous flux Li is exposed to an eye E to be examined with the pupil Ep of the eye E to be examined being widely dilated (pupil dilation). Then, a waveform analysis is performed based on a light-receiving result of the reflected luminous flux Lr, so that the refractive state containing all of the actual optical elements in the region Ae where the reflected luminous flux Lr is transmitted with respect to the widely-dilated pupil Ep1 is calculated. For example, the refractive state at this time is set as a refractive state in the nighttime.

Secondly, as shown in FIG 6B, the wavefront analysis is performed based on, out of the light-receiving results of the reflected luminous flux Lr with respect to the widely-dilated pupil Ep1, the light-receiving result only in the region equivalent to the contracted estimated pupil Ep2 in the daytime, so that the refractive state with respect to the estimated pupil Ep2 is calculated. This means that in the eye E to be examined, the region corresponding to the estimated pupil Ep2 (the region where the reflected luminous flux is transmitted with respect to the estimated pupil Ep2) is cut out from the region Ae where the reflected luminous flux Lr actually is transmitted with respect to the pupil Ep1. Accordingly, it is thought to be capable of obtaining the analysis result (refractive state) similar to that obtained in a case where the wavefront analysis is performed based on the actual light-receiving result of the reflected luminous flux from the eye E to be examined in the state having the estimated pupil Ep2. Here, the contracted estimated pupil Ep2 in the daytime can be obtained by acquiring the diameter dimension d of the pupil Ep in the daytime in advance to estimate the estimated pupil Ep2 as a circle having the diameter dimension d using the center position c of the widely-dilated pupil Ep1 as the center thereof. This diameter dimension d may use the measurement result of the eye E to be examined in brightness to be considered as is in the daytime (in the daytime environment) or may use an average diameter dimension of the pupil in the daytime environment.

However, in the actual eye to be examined, when the pupil is widely dilated or contracted to be small, the center position of the pupil may be displaced or the shape of the pupil may change. For example, as shown in FIG 6B, if it is assumed that the center position in a state where the pupil is widely dilated in a substantially circular shape is in the position shown by the reference sign c in the eye to be examined, the pupil is distorted from the substantially circular shape as shown by the reference sign Ep3 when the pupil is actually contracted, and the center position c' thereof is also displaced from the center position c. For this reason, in the above-described conventional method, the calculated refractive state becomes one different from the actual refractive state in the contracted pupil in the daytime. Here, the displacement of the center position of the pupil or the aspect of the change in the pupil shape varies depending on the eye to be examined. Accordingly, it is difficult that the contracted estimated pupil Ep2 in the daytime as the estimated result is caused to be equal to the actual contracted pupil Ep3 in the daytime.

### (Effects of the ophthalmologic apparatus of the invention of the present application)

As described above, in the ophthalmologic apparatus 10 of the invention of the present application, the light source 81 of the fixation optical system 80 which emits visible light for causing an eye E to be examined to observe a fixation target image is adapted to be capable of changing a light intensity under the control of the control arithmetic unit 11 based on the operation of the light intensity change-over switch 12a of the input unit 12. Accordingly, the light source 81 can expose the eye E to be examined to brightness equivalent to that in the daytime and also to brightness equivalent to that in the nighttime.

For this reason, in the ophthalmologic apparatus 10, the light source 81 of the fixation optical system 80 illuminates the eye E to be examined with a light intensity exposing the eye E to be examined to brightness equivalent to that in the nighttime environment by the operation of the light intensity change-over switch 12a. At the same time, for example, the measurement of the wavefront aberration is performed by the operation of the measurement start switch 12b, so as to be capable of calculating the refractive state containing all of the actual optical elements in the region Ae where the reflected luminous flux Lr is transmitted with respect to a state where the pupil Ep of the eye E to be examined is widely dilated (see reference sign Ep1 in FIG 6A) as shown in FIG 8.

Also, the light source 81 of the fixation optical system 80 illuminates the eye E to be examined with a light intensity exposing the eye E to be examined to brightness equivalent to that in the daytime environment by the operation of the light intensity change-over switch 12a. At the same time, for example, the measurement of the wavefront aberration is performed by the operation of the measurement start switch 12b, so as to be capable of calculating the refractive state containing all of the actual optical elements in the region Ar where the reflected luminous flux Lr is transmitted with respect to a state where the pupil Ep of the eye E to be examined is contracted (see reference sign Ep3 in FIG 6B) as shown in FIG 7. Here, as described above, the center positions of the widely-dilated pupil Ep1 and the contracted pupil Ep3 are misaligned from each other (see reference sign c and reference sign c' in FIG 6B). However, when the wavefront aberration is measured, the fixation direction of the eye E to be examined is adjusted by the observation of the fixation target which is performed by the fixation optical system 80. Accordingly, the eyeball is rotated around the center of the rotation in the eye E to be examined, so that the center position (reference sign c and reference sign c' in FIG 6B) is positioned on the measurement optical axis. Thus, the wavefront aberration can be properly measured without changing the measurement optical axis (see, FIG 7).

As described above, in the ophthalmologic apparatus 10 of the invention of the present application, the light intensity of the light source 81 of the fixation optical system 80 is intentionally changed while the wavefront aberration is measured, more specifically, the eye E to be examined is illuminated with visible light having brightness in the nighttime environment while the wavefront aberration is measured and also the eye E to be examined is illuminated with visible light having brightness in the daytime environment while the wavefront aberration is measured. Accordingly, the refractive state containing all of the actual optical elements in the region Ae (see FIG 8) where the reflected luminous flux Lr is transmitted with respect to the pupil Ep1 (see FIG 6A) which is widely dilated in response to the brightness in the nighttime can be calculated and also the refractive state containing all of the actual optical elements in the region Ar (see FIG 7) where the reflected luminous flux Lr is transmitted with respect to the pupil Ep3 (see FIG 6B) which is contracted in response to the brightness in the daytime can be calculated. For this reason, the refractive state of the actual eye E to be examined under the environments having different brightness regardless of the displacement of the center positions of the pupil Ep or the change in the shape of the pupil Ep, which is caused when the pupil Ep is widely dilated or is contracted to be small. In particular, in Embodiment 1, the actual refractive state of the eye E to be examined in the nighttime environment and the actual refractive state of the eye E to be examined in the daytime environment can be properly measured, which can respectively greatly contribute to proper prescriptions for eyeglasses and contact lenses most suitable for use in the nighttime and for eyeglasses and contact lenses most suitable for use in the daytime.

Additionally, in the ophthalmologic apparatus 10 in the invention of the present application, when the eye E to be examined is illuminated with visible light having the brightness in the nighttime environment while the wavefront aberration is measured, the analysis processing unit 11c calculates the center position c (see FIG 6A) of the widely-dilated pupil Ep1 based on the light-receiving signal S7 from the alignment observation optical system 60, and when the eye E to be examined is illuminated with visible light having the brightness in the daytime environment while the wavefront aberration is measured, the analysis processing unit 11c calculates the center position c' (see FIG 6B) of the contracted pupil Ep3 based on the light-receiving signal S7 from the alignment observation optical system 60. These calculation results can be stored in the storage unit 11e as needed, and can be displayed in the display unit 13 as needed under the control of the image display control unit 11d. Also, in the ophthalmologic apparatus 10, the analysis processing unit 11c analyzes the light-receiving signal S7 from the area sensor 61 of the alignment observation optical system 60, so that the shape of the pupil Ep of the eye E to be examined can be obtained and can be displayed in the display unit 13 as needed under the control of the image display control unit 11d. For this reason, in the ophthalmologic apparatus 10, information on the center position and shape of the pupil Ep which is widely dilated or contracted to be small can be acquired.

Note that it is assumed that the above-described Embodiment 1 has the configuration in which the light intensity of the light source 81 can be switched over in four-level brightness (by the operation of the light intensity change-over switch 12a), including one illuminating the eye E to be examined with visible light having the brightness in the nighttime environment and one illuminating the eye E to be examined with visible light having the brightness in the daytime environment. However, as long as they are two or more values which are intentionally set to be different (the different values in the viewpoint that the substantial change in the optical characteristic of the eye E to be examined may be caused due to the change in the pupil diameter dimension), the actual refractive state of the eye E to be examined under the environments having different brightness can be measured. Thus, the configuration is not limited to the above-described Embodiment 1.

### [Modification 1 of Embodiment 1]

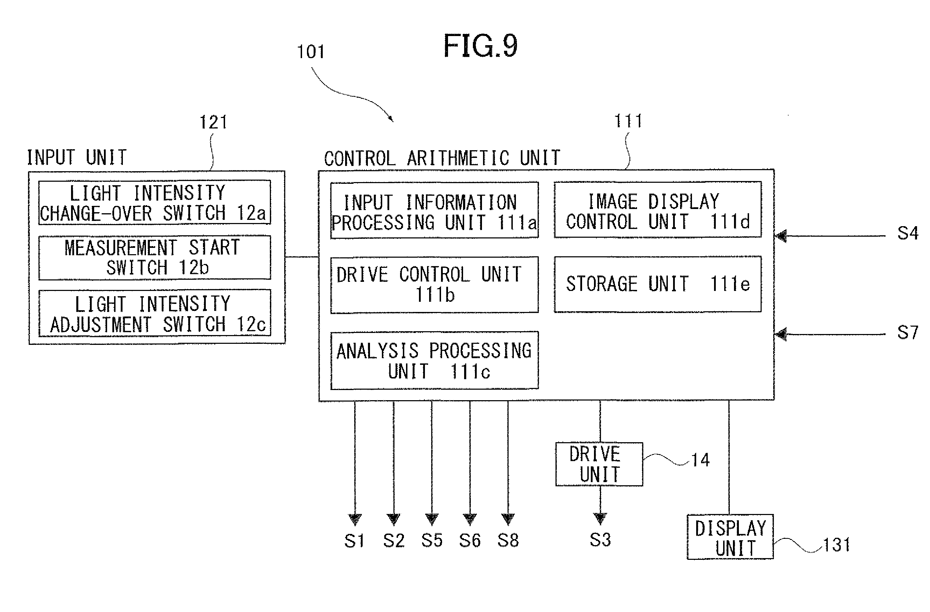

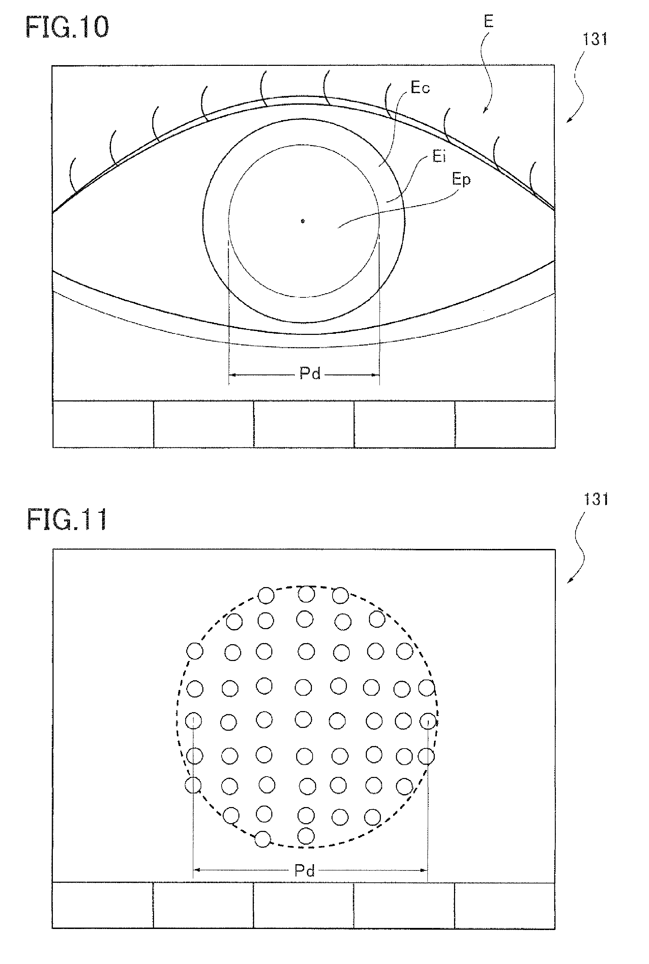

Hereinafter, an ophthalmologic apparatus 101 according to Modification of Embodiment 1 is described. The ophthalmologic apparatus 101 of Modification 1 (see FIG 9) has a basis configuration similar to that of the ophthalmologic apparatus 10 of Embodiment 1, except that the contents of display control performed by an image display control unit 111d of a control arithmetic unit 111 on a display unit 131 are different, and accordingly, the configuration of an input unit 121 is different. Accordingly, same reference signs are given to denote same functional portions and the detailed description thereof is omitted. Note that FIG 9 is an explanatory diagram similar to that of FIG 2 showing an electronic control system of the ophthalmologic apparatus 101 according to Modification 1. Also, FIG 10 is an explanatory diagram showing one example in which a size of the pupil diameter (which is the diameter dimension of the pupil and hereinafter referred to as a pupil diameter dimension Pd) is displayed in the display unit 131 in real time. FIG 11 is an explanatory diagram showing another example in which the pupil diameter dimension Pd is displayed in the display unit 131 in real time.

As shown in FIG 9, in the ophthalmologic apparatus 101, the input unit 121 is provided with a light intensity adjustment switch 12c in addition to a light intensity change-over switch 12a and a measurement start switch 12b. This light intensity adjustment switch 12c is an operation switch for continuously changing the light intensity of the light source 81. For this reason, the light intensity adjustment switch 12c functions as an adjustment operation unit to adjust the light intensity of visible light illuminating means.

In the ophthalmologic apparatus 101, as shown in FIG 10, the image display control unit 111d of the control arithmetic unit 111 causes the display unit 131 to display an image of the eye E to be examined in real time (immediately) and a pupil diameter dimension Pd over the image. The pupil diameter dimension Pd can be calculated in such a manner that the analysis processing unit 111c analyzes a light-receiving signal S7 from the area sensor 61 of the alignment observation optical system 60, and based on the calculation result, the image display control unit 111d causes the display unit 131 to display the pupil diameter dimension Pd.

In the ophthalmologic apparatus 101, when a wavefront aberration is measured, an examiner illuminates the eye E to be examined with the light source 81 of the fixation optical system 80 and the light intensity adjustment switch 12c of the input unit 121 is operated while visually checking the display unit 131. Accordingly, the diameter dimension of the pupil Ep of the eye E to be examined (pupil diameter dimension Pd) can be adjusted, and the measurement start switch 12b is operated once the pupil diameter dimension becomes a desired pupil diameter dimension Pd, so as to be capable of calculating the refractive state containing all of the actual optical elements in the region where the reflected luminous flux is transmitted with respect to the eye E to be examined which is set as a desired pupil diameter dimension Pd.

Also, the eye E to be examined is caused to observe the fixation target image, and the light intensity adjustment switch 12c of the input unit 121 is operated while visually checking the display unit 131, so that the diameter dimension of the pupil Ep of the eye E to be examined (pupil diameter dimension Pd) can be adjusted. Accordingly, utilizing the calculation function of the center position of the pupil Ep similar to that of Embodiment 1, the center position of the pupil Ep when the pupil diameter dimension Pd is a desired one can be measured.

Note that in Modification 1, the pupil diameter dimension Pd is calculated based on the light-receiving signal S7 from the area sensor 61 of the alignment observation optical system 60, and the pupil diameter dimension Pd is superimposed on the image of the eye E to be examined based on the light-receiving signal S7. However, as long as the information of the pupil diameter dimension Pd is displayed in the display unit 13 for allowing the examiner to easily recognize, the configuration is not limited to Modification 1. For example, the pupil diameter dimension Pd is calculated based on the light-receiving signal S4 from the area sensor 31 of the light-receiving optical system 30, and as shown in FIG 11, the pupil diameter dimension Pd may be superimposed on a Hartmann image based on the light-receiving signal S4. The reason is as follows: in the optical system of the ophthalmologic apparatus 101, a Hartmann plate 32 of the light-receiving optical system 30 and the pupil Ep of the eye E to be examined have a conjugated positional relationship, accordingly, the pupil diameter dimension Pd and a range of the Hartmann image (point image) projected on the area sensor 31 by a divided luminous flux which has passed through the Hartmann plate 32 have a constant correlation regardless of the degree of the eye E to be examined, and thus the pupil diameter dimension Pd can be obtained from a circumcircle of the Hartmann image.

### [Modification 2 of Embodiment 1]

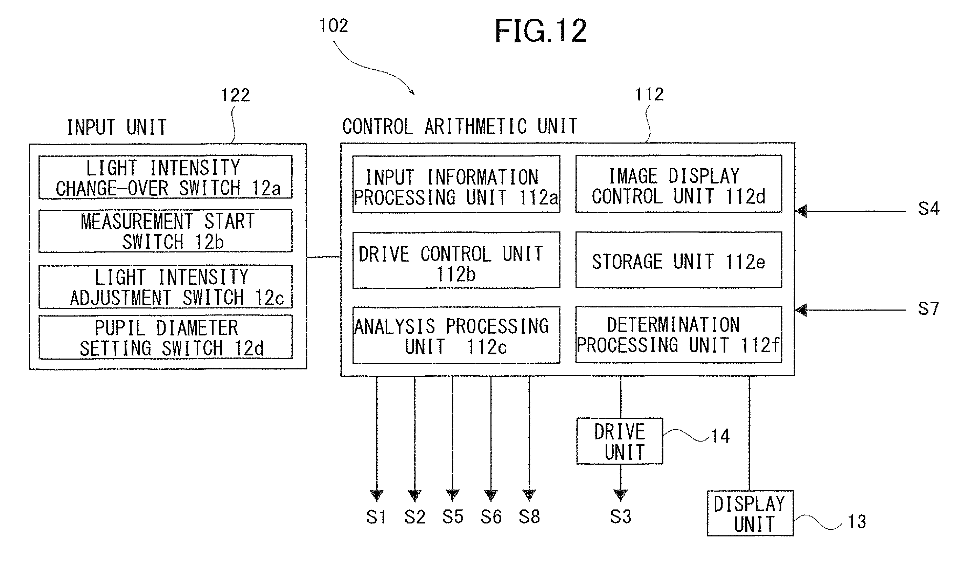

Hereinafter, an ophthalmologic apparatus according to Modification 2 of Embodiment 1 is described. An ophthalmologic apparatus 102 (see FIG 12) of Modification 2 is an example in which a wavefront aberration is automatically measured under the control of a control arithmetic unit 112. The ophthalmologic apparatus 102 of Modification 2 has a basic configuration similar to that of the ophthalmologic apparatus 10 of the Embodiment 1. Thus, same reference signs are given to denote same functional portions as those of Embodiment 1, and the description thereof is omitted. Note that FIG 12 is an explanatory diagram similar to that of FIG 2 showing an electronic control system of the ophthalmologic apparatus 102 according to Modification 2.

As shown in FIG 12, in the ophthalmologic apparatus 102, an input unit 122 is provided with a pupil diameter setting switch 12d in addition to a light intensity change-over switch 12a, a measurement start switch 12b, and a light intensity adjustment switch 12c. This pupil diameter setting switch 12d is an operation switch for inputting a pupil diameter dimension Pd (see FIG 10 and FIG 11) so as to be a reference for automatically measuring a wavefront aberration under the control of the control arithmetic unit 112. A pupil diameter set value set by the operation over the pupil diameter setting switch 12d is stored in a storage unit 112e after being processes as needed by the input information processing unit 112a of the control arithmetic unit 112.

In the ophthalmologic apparatus 102, the control arithmetic unit 112 is provided with a determination processing unit 112f in addition to an input information processing unit 112a, a drive control unit 112b, an analysis processing unit 112c, and an image display control unit 112d.

This determination processing unit 112f acquires the pupil diameter set value stored in the storage unit 112e and information of the pupil diameter dimension in a current state which is calculated based on the light-receiving signal S7 from the area sensor 61 of the alignment observation optical system 60, and sends a signal corresponding to a result of the comparison between the pupil diameter set value and the pupil diameter dimension to the drive control unit 112b. This comparison result includes three kinds of cases where the pupil diameter dimension in the current state is smaller than the pupil diameter set value, where the pupil diameter dimension in the current state is larger than the pupil diameter set value, and where the pupil diameter set value is equal to the pupil diameter dimension in the current state.

When the pupil diameter dimension in the current state is smaller than the pupil diameter dimension set value, the drive control unit 112b which received the signal from the determination processing unit 112f sends a control signal S8 to decrease a light intensity of the light source 81 to the light source 81. When the pupil diameter dimension in the current state is larger than the pupil diameter dimension set value, the drive control unit 112b sends a control signal S8 to increase a light intensity of the light source 81 to the light source 81. When the pupil diameter dimension in the current state is equal to the pupil diameter dimension set value, a wavefront aberration is executed.

In this ophthalmologic apparatus 102, when the measurement of the wavefront aberration is performed, an examiner operates the pupil diameter setting switch 12d of the input unit 122 to set a desired pupil diameter, so that the refractive state containing all of the actual optical elements in the region where the reflected luminous flux is transmitted with respect to the eye E to be examined having the set desired pupil diameter.

Also, the pupil diameter setting switch 12d of the input unit 122 is operated to set a desired pupil diameter dimension and the fixation target image is caused to be observed by the eye E to be examined, so that the diameter dimension of the pupil Ep of the eye E to be examined can be set to be a desired value. Accordingly, utilizing the calculation function of the center position of the pupil Ep similar to that of the Embodiment 1, the center position of the pupil Ep having the desired pupil diameter dimension can be measured.

As described above, the embodiments for implementing an ophthalmologic apparatus according to the invention have been described. However, the present invention is not limited to the above-described embodiments for implementing the invention, and can be modified as needed within a scope without departing from the contents thereof.

Note that, for example, in the above-described embodiment 1, the light-receiving optical system 30 for measuring the wavefront aberration is used as a measurement optical system to measure a distribution of the optical characteristic within the pupil diameter of the eye E to be examined. However, it is only needed to measure the distribution of the optical characteristic of the eye E to be examined by illuminating the ocular fundus Ef of the eye E to be examined with spot light and receiving the luminous flux having passed through the pupil E of the eye E to be examined after being reflected by the ocular fundus Ef, and the configuration is not limited to the configuration of the Embodiment 1.

Also, for example, in the above-described Embodiment 1, the fixation optical system 80 (the light source thereof) is utilized as the visible light illuminating means. However, as long as the eye E to be examined is illuminated with visible light along the optical axis of the measurement optical system, the visible light illuminating means may be provided separately from the fixation optical system 80. Thus, the configuration is not limited to the configuration of the Embodiment 1.

### [Cross-Reference to Related Applications]

This application claims priority based on Japanese Patent Application No. 2009-105753 filed on April 24, 2009 in Japan Patent Office, all of disclosed matters of which are incorporated herein as reference.

## Claims

1. An ophthalmologic apparatus including a measurement optical system capable of measuring distribution of an optical characteristic within a pupil diameter of an eye to be examined,
the ophthalmologic apparatus comprising visible light illuminating means for illuminating the eye to be examined with visible light along an optical axis of the measurement optical system, wherein
the visible light illuminating means is configured to be capable of changing an illuminating light intensity in a range including at least from a light intensity capable of exposing the eye to be examined to brightness equivalent to that in a nighttime environment to a light intensity capable of exposing the eye to be examined to brightness equivalent to that in a daytime environment, and
the measurement optical system is capable of measuring the distribution of the optical characteristic of the eye to be examined which is illuminated by the visible light illuminating means with the brightness equivalent to that in the nighttime environment and is also capable of measuring the distribution of the optical characteristic of the eye to be examined which is illuminated by the visible light illuminating means with the brightness equivalent to that in the daytime environment.

2. The ophthalmologic apparatus according to claim 1, wherein the visible light illuminating means is a fixation optical system to project a fixation target image onto the eye to be examined.

3. The ophthalmologic apparatus according to claim 1 or 2, wherein the visible light illuminating means is configured to be capable of changing the light intensity between two or more preset values which are intentionally made different, the values including the light intensity capable of exposing the eye to be examined to brightness equivalent to that in the nighttime environment and the light intensity capable of exposing the eye to be examined to brightness equivalent to that in the daytime environment.

4. The ophthalmologic apparatus according to claim 1 or 2, further comprising:
a display unit capable of displaying measurement information on the eye to be examined;
an adjustment operation unit to adjust the light intensity of the visible light illuminating means; and
pupil diameter dimension measuring means for measuring a pupil diameter dimension of the eye to be examined, wherein
the pupil diameter dimension is immediately displayed in the display unit.

5. The ophthalmologic apparatus according to claim 1 or 2, further comprising:
pupil diameter dimension measuring means for measuring a pupil diameter dimension of the eye to be examined; and
a control unit to control the measurement optical system and the visible light illuminating means, wherein
the control unit changes the light intensity of the visible light illuminating means, and, once the pupil diameter dimension becomes a predetermined size, causes the measurement optical system to execute measurement of the distribution of the optical characteristic of the eye to be examined.

6. The ophthalmologic apparatus according to any one of claims 1 to 5, wherein the measurement optical system illuminates an ocular fundus of the eye to be examined with spot light and receives a luminous flux which has passed through the pupil of the eye to be examined after being reflected by the ocular fundus, thereby measuring the distribution of the optical characteristic of the eye to be examined.

7. The ophthalmologic apparatus according to any one of claims 1 to 5, wherein the measurement optical system measures a wavefront aberration, thereby measuring the distribution of the optical characteristic of the eye to be examined.

8. The ophthalmologic apparatus according to any one of claims 1 to 7, wherein the optical characteristic of the eye to be examined includes at least a refractive state.

9. The ophthalmologic apparatus according to any one of claims 1 to 7, comprising center position measuring means for measuring the center position of the pupil of the eye to be examined.

10. An ophthalmologic apparatus including a measurement optical system capable of measuring distribution of an optical characteristic within a pupil diameter of an eye to be examined,
the ophthalmologic apparatus comprising:
visible light illuminating means for illuminating the eye to be examined with visible light along an optical axis of the measurement optical system; and
center position measuring means for measuring the center position of the pupil of the eye to be examined, wherein
the visible light illuminating means is configured to be capable of changing an illuminating light intensity in a range including at least from a light intensity capable of exposing the eye to be examined to brightness equivalent to that in a nighttime environment to a light intensity capable of exposing the eye to be examined to brightness equivalent to that in a daytime environment, and
the center position measuring means is capable of measuring the center position of the pupil of the eye to be examined illuminated by the visible light illuminating means with the brightness equivalent to that in the nighttime environment and is also capable of measuring the center position of the pupil of the eye to be examined illuminated by the visible light illuminating means with the brightness equivalent to that in the daytime environment.
